# EUROPEAN PATENT SPECIFICATION

(11) **EP 2 386 549 B1**
(45) Date of publication and mention of the grant of the patent: **19.03.2014**
(21) Application number: 10162440.1
(22) Date of filing: 10.05.2010
(51) Int. Cl.: C07D 333/16

(54) **Process for preparing (S)-(+)-N-methyl-3-(1-naphthyloxy)-3-(2-thienyl)propylamine by using an optically active methylhydroxylaminopropanol compound as intermediate**
Verfahren zur Herstellung von (S)-(+)-N-Methyl-3-(1-naphthyloxy)-3-(2-thienyl)-propylamin mittels einer optisch aktiven Methylhydroxylaminopropanol-Verbindung als Zwischenprodukt
Procédé de préparation de (S)-(+)-N-méthyl-3-(1-naphthyloxy)-3-(2-thienyl)-propylamine en utilisant un composant de méthylhydroxylaminopropanol optiquement actif en tant qu'intermédiaire

(43) Date of publication of application: 16.11.2011
(73) Proprietor: SCI Pharmatech, Inc., Taoyuan (TW)
(72) Inventor: Chen, Bo-Feng, Taoyuan (TW); Lu, Feng-Ju, Taoyuan (TW); Yeh, Jinun-Ban, Taoyuan (TW); Wong,, Weichyun, Toayuan (TW)
(74) Representative: Jones, Helen M.M.

(56) References cited:
- WO-A2-2009/074883
- US-A1- 2009 112 001

## Description

### Background Of The Invention

### 1. Field of the Invention

The present invention relates to a process for preparing (S)-(+)-N-methyl-3-(1-naphthyloxy)-3-(2-thienyl)propylamine and its pharmaceutically acceptable salts with high enantiomeric excess (ee) and high chemical purity by using chiral compounds as an intermediate.

### 2. Description of Related Art

(S)-(+)-N-methyl-3-(1-naphthyloxy)-3-(2-thienyl)propylamine (Duloxetine - trade mark) hydrochloride salt is used as an antidepressant for medical therapy. There are various processes for preparing Duloxetine. For example, U.S. Patent No. 7,538,232 discloses a process for preparation of Duloxetine by reacting (S)-3-methylamino-1-(2-thienyl)propan-1-ol and 1-fluoronaphthalene with potassium hydroxide in a mixed solvent system (i.e., DMSO and toluene) in order to preserve chiral integrity. However, due to different types of organic solvents used in the process for the preparation of Duloxetine, cost of subsequent treatment increases. In addition, the usage of mixed organic solvents is disadvantageous to environmental protection. Therefore, there is still a need to provide a process for preparing chiral Duloxetine.

### Summary Of The Invention

The present invention provides a simple and safe process for preparing Duloxetine with high purity and quality. Particularly, the present disclosure provides a compound of formula (II)used in a process for preparing Duloxetine 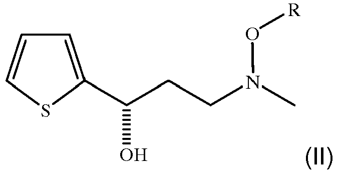 wherein R is a hydrogen atom, C₁-₈ alkyl or C₆₋₁₀ aryl, and the absolute configuration of a chiral center thereof is S.

In one aspect, the present invention provides use of the compound of formula (II) as an intermediate in preparation of (S)-(+)-N-methyl-3-(1-naphthyloxy)-3-(2-thienyl)propylamine (Duloxetine). The process is defined in claim 1.

In another aspect, the present invention provides a process for preparing (S)-(+)-N-methyl-3-(1-naphthyloxy)-3-(2-thienyl)propylamine (Duloxetine) with higher yield and lower cost by using (S)-3-methylamino-1-(2-thienyl)-propan-1-ol, which has a structure of formula (III) and is produced from the intermediate of formula (II).

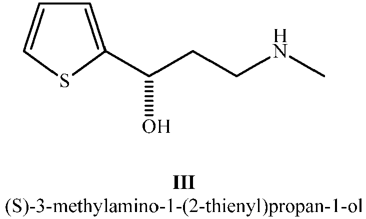

In another aspect the present invention provides a process for preparing (S)-(+)-N-methyl-3-(1-naphthyloxy)-3-(2-thienyl)propylamine represented by the following formula, 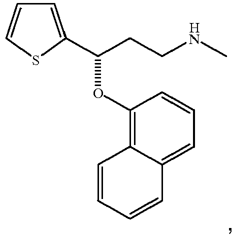 comprising reacting (S)-(-)-3-methylamino-1-(2-thienyl)propan-1-ol with KOR¹ and excess 1-halonaphthalene in DMSO, wherein R¹ is C₁₋₆ alkyl; and halo is F, Cl, Br or I, and wherein DMSO is used in amounts ranging from one to five times the amount of (S)-(-)-3-methylamino-1-(2-thienyl)propan-1-ol.

In a preferred embodiment of both process aspects, the process for preparing Duloxetine is summarized in the following scheme 1 and includes steps of: (i) performing a Mannich reaction of 2-acetylthiophene, formaldehyde and a compound represented by formula HNCH₃(OR) to form a compound represented by formula (I); (ii) enantioselectively reducing the compound represented by formula (I) to a compound represented by formula (II); (iii) performing an N,O-cleavage reaction on the compound represented by formula (II) to give (S)-(-)-3-methylamino-1-(2-thienyl)-propan-1-ol as shown by formula (III); and (iv) reacting the (S)-(-)-3-methylamino-1-(2-thienyl)-propan-1-ol with excess 1-halonaphthalene to form (S)-(+)-N-methyl-3-(1-naphthyloxy)-3-(2-thienyl)propylamine (Duloxetine), wherein R is a hydrogen atom, C₁₋₈ alkyl or C₆₁₀ aryl, preferably C₁₋₄ alkyl, and more preferably methyl; and halo is F, CI, Br or I.

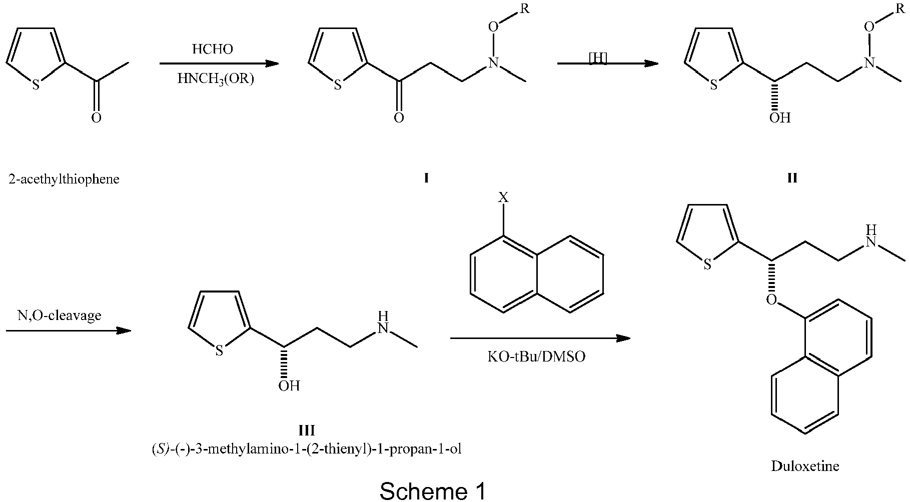

Further, in the step (iv) of the process of the present inventions, KOR¹ and DMSO are used in the naphthalenation reaction, wherein R¹ is C₁₋₆ alkyl, preferably butyl, and more preferably tert-butyl, and wherein DMSO is used in amounts ranging from one to five times the amount of (S)-(-)-3-methylamino-1-(2-thienyl)propan-1-ol.

### Detailed Description Of Preferred Embodiments

The following illustrative embodiments are provided to illustrate the disclosure of the present invention. These and other advantages and effects can be apparently understood by those in the art after reading the disclosure of this specification.

The present disclosure provides a compound represented by formula (II) in optical active form used in a process for preparing Duloxetine: 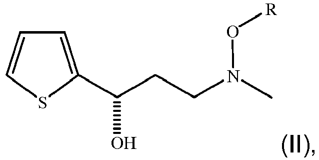 wherein R is a hydrogen atom, C₁₋₈ alkyl or C₆₋₁₀ aryl, preferably C₁₋₄ alkyl, and more preferably methyl. Furthermore, an absolute configuration of a chiral center of the compound is S.

Furthermore, the present invention provides a process for preparing (S)-(+)-N-methyl-3-(1-naphthyloxy)-3-(2-thienyl)propylamine (Duloxetine), wherein the compound represented by formula (II) is used as an intermediate. The process of the present invention is summarized in Scheme 1.

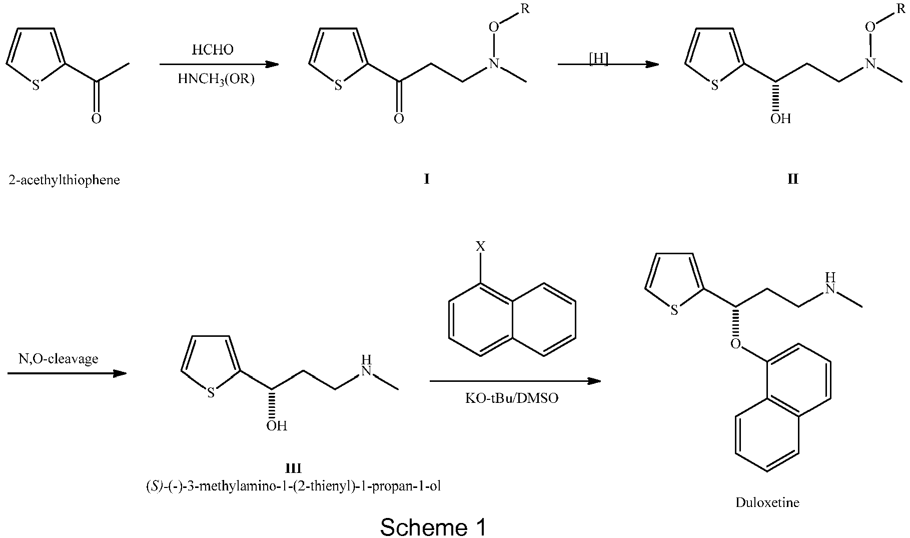

In the above Scheme 1, R is a hydrogen atom, C₁₋₈ alkyl or C₆₋₁₀ aryl, preferably C₁₋₄ alkyl, and more preferably methyl; halo is F, CI, Br or I; and R¹ is C₁₋₆ alkyl, preferably butyl, and more preferably tert-butyl.

In more details, the process includes steps of:
performing a Mannich reaction of 2-acetylthiophene, formaldehyde and a compound represented by formula HNCH₃(OR) to form a compound represented by formula (I);
enantioselectively reducing the compound represented by formula (I) to a compound represented by formula (II);
performing an N,O-cleavage reaction on the compound of formula (II) to form (S)-(-)-3-methylamino-1-(2-thienyl)-propan-1-ol as shown by formula (III); and
reacting the (S)-(-)-3-methylamino-1-(2-thienyl)propan-1-ol with excess 1-halonaphthalene to form (S)-(+)-N-methyl-3-(1-naphthyloxy)-3-(2-thienyl) propylamine (Duloxetine).

The step (i) of the process is carried out at a temperature in the range of from 15°C to 90°C, preferably from 40°C to 80°C, and more preferably from 50°C to 70°C. The compound of formula (I) obtained in the step (i) is either as a free form or as an acid addition salt.

The reduction of the compound represented by formula (I) in the step (ii) is asymmetric reduction, and preferably chiral reduction. Hence, an optically active form of the compound represented by formula (II) is obtained. The optically active form can be obtained via asymmetric hydrogenation using a catalyst with chiral ligands, or a hydride with chiral ligands. In the step (ii), a chiral reducing agent used in the chiral reduction is selected from the group consisting of complex hydride, borane, transition metal catalyst and microbial dehydrogenase.

The process of forming the compound of formula II by asymmetric reduction of a compound of formula I forms a further aspect of the present invention. In this aspect the compound of formula I may be made by step (i) defined above.

In one preferred embodiment of the processes involving reaction of the compound of formula I to form a compound of formula II, the reduction of the compound of formula (I) in the step (ii) is carried out in a mixture of an alcohol (such as methanol) and a base (such as potassium tert-butoxide), and in the presence of a catalyst that includes an enantiomer-enriched bidentate phosphorus-containing ligand, a transition metal and a diamine, preferably a chiral diamine. An example of the catalyst is RuCl₂((R)-3,5-xylylBINAP)((2R)-DAIPEN). The reaction mixture is hydrogenated at predetermined pressure to yield the compound of formula (II) with a high ee value.

In the step (iii) of the process of the present invention, the N,O-cleavage reaction of the compound represented by formula (II) is carried out by hydrogenation in the presence of a catalyst such as Raney-nickel, or by chemical reduction processes such as those by using LiAIH₄ or zinc metal as a reducing agent.

In the step (iv) of the process of any aspect of the present invention, the reaction of (S)-(-)-3-methylamino-1-(2-thienyl)propan-1-ol and excess 1-halonaphthalene is carried out by using an appropriate base, such as potassium tert-butoxide in a suitable amount of DMSO to form (S)-(+)-N-methyl-3-(1-naphthyloxy)-3-(2-thienyl)propylamine (Duloxetine). The excess amount of 1-halonaphthalene, ranging from about 1.5 to 4 equivalents, can be recovered. The reaction is performed at a temperature in the range of from 20°C to 110°C, preferably from 40°C to 90°C, for 1 to 24 hours. The present invention is conducted in the presence of excess 1-halonaphthalene and a suitable amount of DMSO to overcome the problems regarding racemization and environmental protection. DMSO is used in amounts ranging from one to five times the amount of (S)-(-)-3-methylamino-1-(2-thienyl)propan-1-ol.

In comparison with the prior art, the process of the present invention provides optically pure Duloxetine with higher yield and lower cost. This process should operate particularly well on an industrial scale regarding to economic and ecological aspects.

### EXAMPLES

### Example 1: Synthesis of 3-methoxymethylamino-1-(2-thienyl)-1-propanone hydrochloride salt

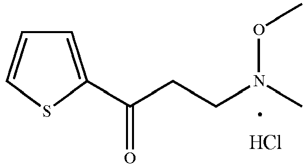

27.7 g of N,O-dimethylhydroxylamine hydrochloride, 9.3 g of paraformaldehyde, 6.4 g of hydrochloride (32%), 30.0 g of 2-acetylthiophene and 100 g of isopropanol were provided into a flask. After being stirred at 60°C for 13 hours, the reaction mixture was cooled down to room temperature. The crystal thus formed was filtered, washed with 30 g of isopropanol and dried under reduced pressure to obtain 42.5 g of 3-methoxymethylamino-1-(2-thienyl)-1-propanone hydrochloride salt (75.9%).
1H NMR (400 MHz, CDCl₃) δ (ppm) = 3.1 (s, 3H), 3.7 - 3.8 (br, 4H), 4.1 (s, 3H), 7.2 (t, J = 4.5 Hz, 1H), 7.7 (d, J = 4.9 Hz, 1H), 7.9 (d, J = 3.5 Hz, 1H).

### Example 2: Synthesis of (S)-3-methoxymethylamino-1-(2-thienyl)propan-1-ol

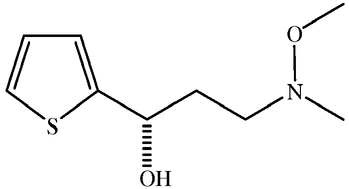

A degassed methanol solution (4 ml) containing RuCl₂((R)-3,5-xylyIBINAP)((2R)-DAIPEN) (10 mg), 3-methoxymethylamino-1-(2-thienyl)-1-propanone (160 mg), potassium tert-butoxide (100 mg) and methanol (10 ml) was charged in a glass autoclave under an argon gas flow. After deaeration and replacement by argon, hydrogen was introduced to a predetermined pressure. The resulting solution was hydrogenated at 20°C for 12 hours. Upon completion of hydrogenation, the reaction mixture was concentrated to give an oily product (161 mg, 95.8% measured by HPLC assay, 95% ee).
1H NMR (400 MHz, CDCl₃) δ (ppm) = 3.0 (s, 3H), 3.0 - 3.1 (m, 1H), 4.1 (s, 3H), 4.0 - 4.1 (m, 3H), 6.1 (dt, J = 7.4, 15.4 Hz, 1H), 6.9 (d, J = 15.7 Hz, 1H), 7.0 (dd, J = 3.7, 5.0 Hz, 1H), 7.1 (d, J = 3.4 Hz, 1H).

### Example 3: Synthesis of (S)-(-)-3-methylamino-1-(2-thienyl)-propan-1-ol

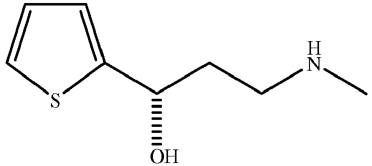

(S)-3-methoxymethylamino-1-(2-thienyl)propan-1-ol obtained from Example 2 was dissolved in 10 ml of methanol with 8 mg of Raney-nickel. This resulting solution was provided in a glass autoclave and hydrogenated at 50°C for 12 hours. Upon completion of hydrogenation, the reaction mixture was filtered, and the solvent was removed under reduced pressure to give a crystal compound (122mg, 90.8% measured by HPLC assay, 95% ee). The crude product was further purified by re-crystallization in toluene to give a product with optical purity as high as 100% ee.

### Example 4: Synthesis of (S)-(+)-N-methyl-3-(1-naphthyloxy)-3-(2-thienyl)propylamine (Duloxetine)

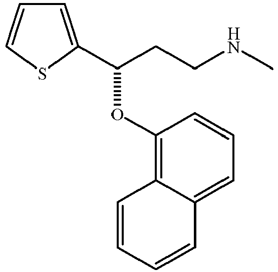

(S)-(-)-3-methylamino-1-(2-thienyl)-propan-1-ol (20.0 g) and 1-fluoronaphthalene (68.3 g) were charged into a 4-neck round bottomed flask. Potassium tert-butoxide (13.1 g) and DMSO (36.0 g) were then added, and the resulting mixture was heated to 60°C for 8 hours. After completion of the reaction, the reaction mixture was cooled down and washed with water. Layers were separated, and the organic layer was further extracted with 32% HCI(aq) (14.7 g) to separate Duloxetine from 1-floronaphthalene. The acidic aqueous layer was adjusted to pH 12-13 with 45% NaOH(aq) (17.6g) to obtain Duloxetine in a free base form as an oily liquid (31.3 g, 90%). Optical purity of the resulting Duloxetine measured by chiral HPLC was 95% e.e.
1H NMR (400 MHz, CDCl₃) δ (ppm) = 2.2 (m, 1H), 2.4 (m, 1H), 2.4 (s, 3H), 2.8 (m, 2H), 5.8 (m, 1H), 6.8 (d, 1H), 6.9 (m, 1H), 7.1 (d, 1H), 7.2 (d, 1H), 7.3 (d, 1H), 7.4 (m, 1H), 7.5 (m, 2H), 7.8 (m, 1H), 8.3 (m, 1H).

The foregoing descriptions of the detailed embodiments are only illustrated to disclose the features and functions of the present invention and not restrictive of the scope of the present invention. Accordingly, the protection sought herein is as set forth in the claims below.

## Claims

1. A process for preparing (S)-(+)-N-methyl-3-(1-naphthyloxy)-3-(2-thienyl)propylamine, comprising the steps of:
performing an N,O-cleavage reaction on a compound represented by formula (II), 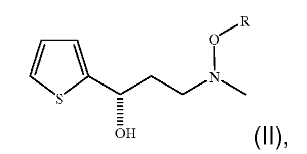
to form (S)-(-)-3-methylamino-1-(2-thienyl)propan-1-ol as shown in formula (III), 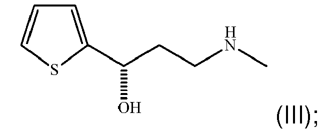 and
reacting the (S)-(-)-3-methylamino-1-(2-thienyl)propan-1-ol with excess 1-halonaphthalene to form (S)-(+)-N-methyl-3-(1-naphthyloxy)-3-(2-thienyl)propylamine represented by the following formula, 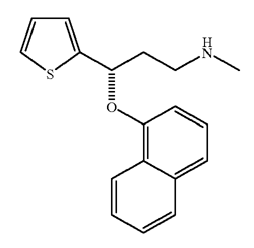 wherein R is a hydrogen atom, C₁₋₈ alkyl or C₆₋₁₀ aryl; and halo is F, CI, Br or I, wherein KOR¹ and DMSO are used in the step of reacting the (S)-(-)-3-methylamino-1-(2-thienyl)propan-1-ol with 1-halonaphthalene, and R¹ is C₁₋₆ alkyl, and wherein DMSO is used in amounts ranging from one to five times the amount of (S)-(-)-3-methylamino-1-(2-thienyl)propan-1-ol.

2. The process of claim 1, wherein R is C₁₋₄ alkyl.

3. The process of claim 2, wherein R is methyl.

4. The process of claim 1, wherein the 1-halonaphthalene is 1-fluoronaphthalene.

5. The process of claim 1, wherein R¹ is tert-butyl.

6. The process of claim 1, wherein the compound of formula II is produced in a preliminary step of enantioselectively reducing a compound of formula I

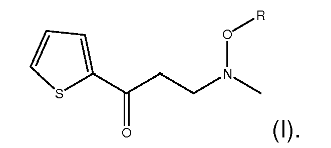

7. The process of claim 6 wherein the compound of formula II is produced using a hydrogenation catalyst having chiral ligands, or a hydride with chiral ligands.

8. The process of claim 7 wherein the compound of formula II is produced in the presence of a catalyst that includes an enantiomer-enriched bidentate phosphorus-containing ligand, a transition metal and a diamine

9. The process of claim 8 wherein the diamine is a chiral diamine.

10. The process of claim 6, wherein the compound of formula I is produced in a preliminary step by the Mannich reaction of 2-acetylthiophene, formaldehyde and the compound HNCH₃(OR) to form the compound of formula I as an acid addition salt or in free base form.

## Patentansprüche

1. Verfahren zur Herstellung von (S)-(+)-N-Methyl-3-(1-naphthyloxy)-3-(2-thienyl)propylamin, umfassend die Schritte von:
Durchführung einer N,O-Spaltungsreaktion an einer durch Formel (II) dargestellten Verbindung, 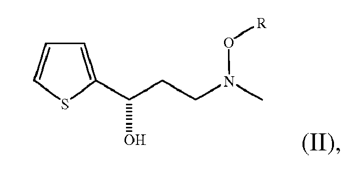
zur Bildung von (S)-(-)-3-Methylamino-1-(2-thienyl)propan-1-ol wie in Formel (III), 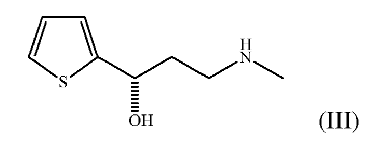 gezeigt, und
zur Reaktion bringen des (S)-(-)-3-Methylamino-1-(2-thienyl)propan-1-ols mit überschüssigem 1-Halogennaphthalin zur Bildung von (S)-(+)-N-Methyl-3-(1-naphthyloxy)-3-(2-thienyl)propylamin, dargestellt durch die folgende Formel, 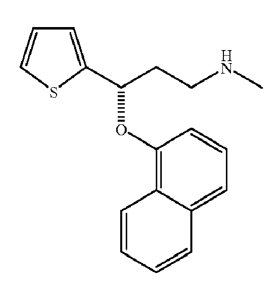 wobei R ein Wasserstoffatom, C₁₋₈-Alkyl oder C₆₋₁₀-Aryl ist; und Halogen F, Cl, Br oder I ist, wobei KOR¹ und DMSO im Schritt des zur Reaktion Bringens des (S)-(-)-3-Methylamino-1-(2-thienyl)propan-1-ols mit 1-Halogennaphthalin verwendet werden, und R¹ C₁₋₆-Alkyl ist, und wobei DMSO in Mengen verwendet wird, die im Bereich von ein- bis fünfmal der Menge von (S)-(-)-3-Methylamino-1-(2-thienyl)propan-1-ol liegen.

2. Verfahren nach Anspruch 1, wobei R C₁₋₄-Alkyl ist.

3. Verfahren nach Anspruch 2, wobei R Methyl ist.

4. Verfahren nach Anspruch 1, wobei das 1-Halogennaphthalin 1-Fluornaphthalin ist.

5. Verfahren nach Anspruch 1, wobei R¹ tert-Butyl ist.

6. Verfahren nach Anspruch 1, wobei die Verbindung der Formel II in einem vorläufigen Schritt der enantioselektiven Reduktion einer Verbindung der Formel I 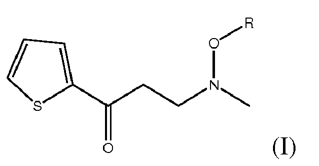 hergestellt wird.

7. Verfahren nach Anspruch 6, wobei die Verbindung der Formel II unter Verwendung eines Hydrierungskatalysators mit chiralen Liganden oder einem Hydrid mit chiralen Liganden hergestellt wird.

8. Verfahren nach Anspruch 7, wobei die Verbindung der Formel II bei Vorliegen eines Katalysators hergestellt wird, der einen enantiomer angereicherten, zweizähnigen, Phosphor enthaltenden Liganden, ein Übergangsmetall und ein Diamin einschließt.

9. Verfahren nach Anspruch 8, wobei das Diamin ein chirales Diamin ist.

10. Verfahren nach Anspruch 6, wobei die Verbindung der Formel I in einem vorläufigen Schritt durch die Mannich-Reaktion von 2-Acetylthiophen, Formaldehyd und der Verbindung HNCH₃(OR) zur Bildung der Verbindung der Formel I als ein Säureadditionssalz oder in freier Basenform hergestellt wird.

## Revendications

1. Procédé de préparation de (S)-(+)-N-méthyl-3-(1 -naphtyloxy)-3-(2-thiényl)propylamine, comprenant les étapes consistant à :
réaliser une réaction de clivage des sites N,O- sur un composant représenté par la formule (II), 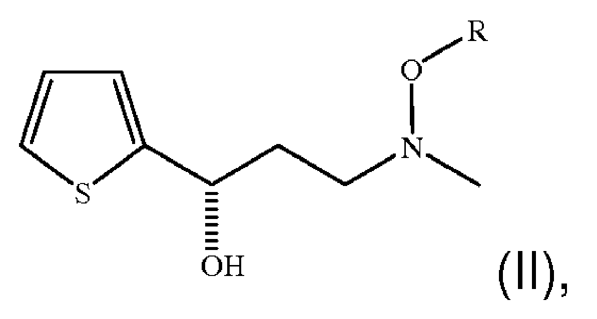
afin de former le (S)-(-)-3-méthylamino-1-(2-thiényl)propane-1-ol comme indiqué dans la formule (III), 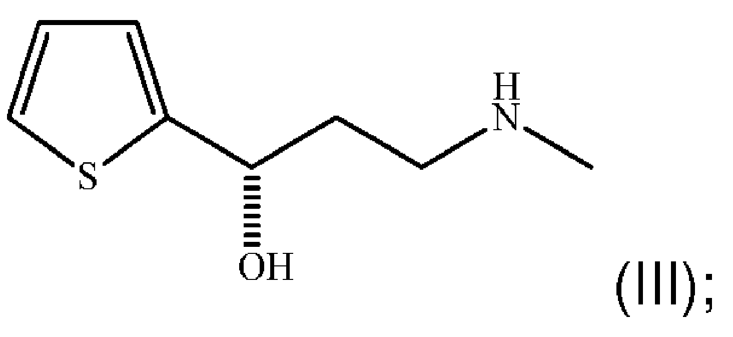 et
faire réagir le (S)-(-)-3-méthylamino-1-(2-thiényl)propane-1-ol avec du 1-halonaphtalène excédentaire pour former le (S)-(+)-N-méthyl-3-(1-naphtyloxy)-3-(2-thiényl)propylamine représenté par la formule suivante, 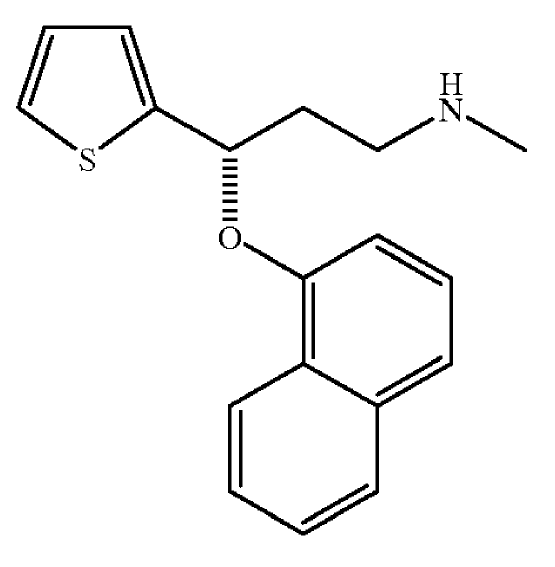 dans laquelle R est un atome d'hydrogène, un alkyle en C₁ à C₈ ou un aryle en C₆ à C₁₀; et halo représente F, CI, Br ou I, dans lequel KOR¹ et le diméthylsulfoxyde (DMSO) sont utilisés dans l'étape de mise en réaction du (S)-(-)-3-méthylamino-1-(2-thiényl)propane-1-ol avec du 1-halonaphtalène et R¹ désigne alkyle en C₁ à C₆ et dans lequel le DMSO est utilisé selon des quantités se situant dans la plage de une à cinq fois la quantité de (S)-(-)-3-méthylamino-1-(2-thiényl)propane-1-ol.

2. Procédé selon la revendication 1, dans lequel R désigne alkyle en C₁ à C₄.

3. Procédé selon la revendication 2, dans lequel R désigne méthyle.

4. Procédé selon la revendication 1, dans lequel le 1-halonaphtalène désigne le 1-fluoronaphtalène.

5. Procédé selon la revendication 1, dans lequel R¹ désigne tert-butyle.

6. Procédé selon la revendication 1, dans lequel le composant de la formule II est produit lors d'une étape préliminaire d'énantiosélectivité en réduisant un composant de la formule I

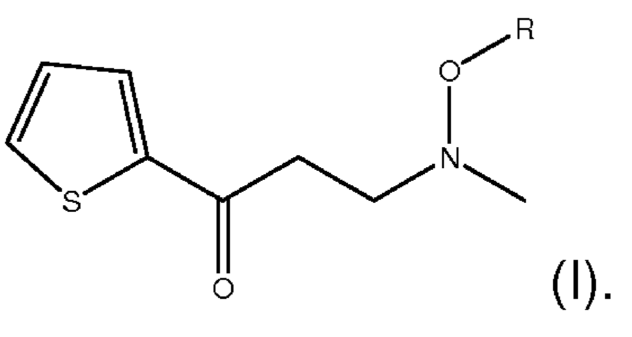

7. Procédé selon la revendication 6, dans lequel le composant de la formule II est produit en utilisant un catalyseur d'hydrogénation comprenant des ligands chiraux ou un hydrure avec des ligands chiraux.

8. Procédé selon la revendication 7, dans lequel le composant de la formule II est produit en présence d'un catalyseur qui comprend un ligand contenant du phosphore bidenté enrichi d'énantiomère, un métal de transition et une diamine.

9. Procédé selon la revendication 8, dans lequel la diamine est une diamine chirale.

10. Procédé selon la revendication 6, dans lequel le composant de la formule I est produit lors d'une étape préliminaire par la réaction de Mannich du 2-acéthylthiophène, du formaldéhyde et du composant HNCH₃(OR) pour former le composant de la formule I en un sel d'addition acide ou sous forme de base libre.
